# EUROPÄISCHE PATENTSCHRIFT

(11) **EP 1 185 548 B1**
(45) Veröffentlichungstag und Bekanntmachung des Hinweises auf die Patenterteilung: **12.04.2006**
(21) Anmeldenummer: 00903580.9
(22) Anmeldetag: 15.01.2000
(51) Int. Cl.: C07K 1/00, B04B 3/00, C07K 14/62, C07K 1/14, C07K 1/36

(54) **VERFAHREN ZUR TROCKNUNG VON PROTEINKRISTALLEN**
METHOD FOR DRYING PROTEIN CRYSTALS
PROCEDE DE SECHAGE DE CRISTAUX PROTEINIQUES

(30) Priorität: 27.01.1999 DE 19903125
(43) Veröffentlichungstag der Anmeldung: 13.03.2002
(73) Patentinhaber: Sanofi-Aventis Deutschland GmbH, 65929 Frankfurt am Main (DE)
(72) Erfinder: DEUSSER, Rolf, D-65843 Sulzbach (DE); KRÄMER, Peter, D-65760 Eschborn (DE); THUROW, Horst, D-65779 Kelkheim (DE)
(86) Internationale Anmeldenummer: PCT/EP2000/000287
(87) Internationale Veröffentlichungsnummer: WO 2000/044767

(56) Entgegenhaltungen:
- EP-A- 0 454 045
- EP-A- 0 622 376
- WO-A-97/44445

## Beschreibung

Proteine sind in vielen handelsüblichen Zubereitungsformen enthalten. Insbesondere sind Proteine als Wirkstoff in einigen pharmazeutischen Arzneimitteln enthalten. Für die Herstellung dieser Zubereitungsformen ist es zweckmäßig, die Proteine in kristalliner Form einzusetzen. Neben der besseren Handhabbarkeit sind insbesondere die Proteine in Form von trockenen Kristallisaten stabiler als z.B. in gelöster Form.

So wird z.B. für die Herstellung von pharmazeutischen Zubereitungen mit dem Hormon Insulin als Wirkstoff das Protein Insulin in kristalliner Form eingesetzt. Kristallisiertes Insulin ist z.B. bei einer Temperatur von -20 °C über mehrere Jahre haltbar.

Bekannt sind kristalline Formen von Proteinen mit einem Molekulargewicht bis zu mehreren Hunderttausend Daltons aber auch von Peptiden mit einem niedrigeren Molekulargewicht. Die Aminosäuresequenz der Proteine kann entweder mit einer in der Natur vorkommenden Sequenz identisch sein oder sie kann gegenüber der natürlichen Form verändert sein. Neben den Ketten von Aminosäuren können die Proteine auch Zucker-Reste oder andere Liganden als Seitenketten enthalten. Die Proteine können aus natürlichen Quellen isoliert worden sein, oder sie können gentechnisch oder synthetisch hergestellt worden sein, oder sie können durch eine Kombination dieser Verfahren gewonnen worden sein.

Proteine zeigen in wäßriger Lösung eine mehr oder weniger komplizierte dreidimensionale Struktur, die auf einer spezifischen räumlichen Faltung der Aminosäureketten beruht. Die intakte Struktur eines Proteins ist für seine biologische Wirkung essentiell. Bei der Kristallisation der Proteine aus wäßrigen Lösungen bleibt diese Struktur weitgehend erhalten. Die Kristallstruktur eines Proteins wird hauptsächlich durch seine Aminosäuresequenz determiniert, daneben aber auch durch Einschlüsse von niedermolekularen Substanzen wie z.B. Metallionensalzen und Wassermolekülen. Insbesondere ist die Anwesenheit einer bestimmten Menge von intrakristallinen Wassermolekülen (Kristallwasser) für die Stabilität der Kristallstruktur von Proteinen notwendig.

So benötigen z.B. lnsulinkristalle einen optimalen Rest-Wassergehalt (etwa zwischen 1 bis 7 %). Hat man durch Übertrocknen der Kristalle einen zu geringen Feuchtegehalt erzielt, so ist bereits Kristallwasser aus den Kristallen entzogen worden. Dadurch ist die chemische Stabilität des Insulins beeinträchtigt, so daß es z.B. zur Bildung von höhermolekularen Verbindungen kommt. Es wird vermutet, daß die höhermolekularen Anteile im Insulin für immunologische Unverträglichkeitsreaktionen verantwortlich sind. Im Extremfall kann das Insulin soweit denaturiert werden, daß die Kristalle in wäßrigen Medien nicht mehr löslich sind. Hat man dagegen beim Trocknen ein Kristallisat mit einem zu hohen Feuchtegehalt erhalten, so befindet sich zu viel Wasser zwischen den einzelnen Kristallen. In diesem interkristallinen Wasser ist das Insulin teilweise gelöst. Die Stabilität von gelöstem Insulin ist aber deutlich geringer als die von festen Formen.

Es ist bekannt, daß man Proteinkristalle, insbesondere Insulinkristalle, trocknen kann, indem man aus einer Kristallsuspension die Kristalle durch Filtration isoliert und den Filterkuchen bei einer Temperatur oberhalb 0 °C im Vakuum trocknet. Es ist auch bekannt, daß man den Trocknungsprozeß beschleunigen kann, indem man vor dem Trocknen das interkristalline Wasser gegen Ethanol austauscht. Während des Trocknens sind die Kristallisate auf Trocknungsblechen in dünner Schicht verteilt oder werden auf dem Filter bewegt.

Bei einem anderen Verfahren werden die Proteinkristalle, beispielsweise Insulinkristalle, als eine wäßrigen Suspension bei einer Temperatur unterhalb 0 °C auf Trocknungsblechen eingefroren und anschließend im Vakuum gefriergetrocknet.

Bei beiden genannten Verfahren ist das Erreichen weder einer definierten noch der optimalen Restfeuchte in den getrockneten Kristallen ausreichend gewährleistet. Bei beiden Verfahren ist der Trocknungsprozeß bis zum Schluß kinetisch kontrolliert und muß rechtzeitig abgebrochen werden, um ein Übertrocknen zu vermeiden. Die Erfahrung zeigt, daß es schwierig ist, den richtigen Zeitpunkt für den Abbruch der Trocknung zu bestimmen. Der resultierende Feuchtegehalt der Kristalle hängt nicht nur von der Schichtdicke auf den Trocknungsblechen ab sondern auch von der Größe der Kristalle (bzw. deren Oberfläche). Da der Kristallisationsprozeß zu Insulinkristallen mit einer mehr oder weniger breiten Größenverteilung führt, besteht das in einem kinetisch kontrollierten Prozeß getrocknete Kristallisat aus einer Mischung von trockneren kleinen und feuchteren großen Kristallen.

Ein weiterer Nachteil dieser Verfahren besteht darin, daß es sehr schwierig ist, die Beschickung und Entleerung der zur Trocknung verwendeten Vorrichtungen zu automatisieren. So ist für den Vorgang der Beschickung und Entleerung noch weitgehend Handarbeit notwendig, die das Risiko einer Kontaminierung mit Keimen und Fremdpartikeln mit sich bringt. So verlangen z.B. die gegenwärtig gültigen Pharmakopoen, daß kristallines Insulin zur Herstellung pharmazeutischer Präparate keimarm aber nicht keimfrei sein muß.

In der europäischen Patentanmeldung EP 0 454 045 A2 ist ein Zentrifugentrockner mit einer horizontal gelagerten Antriebswelle, einer daran mitdrehend angeschlossenen Trommel, einem innerhalb der Trommel angeordneten Filter, der einen von der Anschlussseite der Antriebswelle aus konisch erweiterten Arbeitsraum umschließt, einer eine Stirnseite des Arbeitsraums bildenden, axial verschiebbaren Stauscheibe, und mit einem Trommel und Stauscheibe kapselnden Zentrifugengehäuse beschrieben, bei dem das Zentrifugiergut durch Fliehkraft entlang dem Filterkonus in gesteuerter Schichtdicke durch den Austrittsspalt in einen das offene Konusende umgebenden Ringkanal gelangt, aus dem es mit am Trommelumfang befestigten Schaufeln und/oder eingeblasenem Fördergas abtransportiert wird.

In der internationalen Patentanmeldung WO 97/44445 wird eine Technologie zur Biokatalyse zur Durchführung selektiver chemischer Reaktionen beschrieben. U.a. werden gecrosslinkte Proteinkristallformulierungen und ihr Gebrauch als Katalysatoren bei chemischen Reaktionen unter Verwendung organischer Lösungsmittel offenbart.

In der europäischen Patentanmeldung EP 0 662 376 A2 wird ein Verfahren zur Herstellung von einer amorphen monophärischen Form von Insulinderivaten beschrieben, wobei man das Insulinderivat in einem n-Propanol-Puffer-Gemisch bei einem pH von 4,5 bis 6,5 löst, einen n-Propanol-Gehalt von mehr als 13 % bezogen auf Wasser einstellt und die erhaltene Lösung anschließend mit Wasser verdünnt.

Im US-Patent 5,526,581 ist ein Verfahren zur Trocknung von Tabak oder anderem hygroskopischem, organischem Material beschrieben, wobei verschiedene Eigenschaften des zu trocknenden Materials, z.B. das Zylindervolumen (CV) oder die Degradation des Materials sich nicht signifikant ändert. Zur Trocknung wird ein Luftstrom eingesetzt, dessen relative Luftfeuchte nahe den Gleichgewichtsbedingungen des zu trocknenden Materials liegt.

Im folgenden wird ein Trocknungsverfahren beschrieben, bei dem alle kritischen Prozeßschritte nacheinander in einer Maschine (Zentrifugentrockner) ablaufen:
1. Abfiltrieren der Kristalle aus einer wäßrigen Suspension.
2. Waschen des Filterkuchens.
3. Austausch der Waschflüssigkeit gegen ein Trocknungsmedium.
4. Trockenschleudem des Filterkuchens.
5. Absprengen des Filterkuchens vom Filter und Umwandlung in ein Wirbelbett.
6. Trocknung der Kristalle im Wirbelbett mit einem angefeuchteten Stickstoffstrom.
7. Entleeren der getrockneten Kristalle mit einem Stickstoff-Druckstoß in einen angeflanschten Behälter.

Demgemäß betrifft die vorliegende Erfindung ein Verfahren zur Trocknung von Proteinkristallen ausgehend von einer wäßrigen Proteinkristallsuspension, welches sich dadurch auszeichnet, daß die Proteinkristallsuspension in einem Zentrifugentrockner getrocknet wird.

Die nachfolgend beschriebenen Versuche wurden in einem handelsüblichen Zentrifugentrockner durchgeführt.

Für den beschriebenen Trocknungsprozeß ist es besonders vorteilhaft, wenn der abgesprengte Filterkuchen in ein Wirbelbett überführt werden kann. Die Versuche haben gezeigt, daß ohne den Austausch des interkristallinen Wassers gegen eines der unten beschriebenen Trocknungsmedien kein Wirbelbett erzeugt werden konnte, sondern ein Gemenge aus mehr oder weniger großen Kristallaggregaten entstand, so daß eine gleichmäßige Trocknung unmöglich wurde.

Andere Versuche haben gezeigt, daß nach einem Austausch des interkristallinen Wassers gegen ein reines nicht-wäßriges Trocknungsmedium, z.B. 100 %iges Ethanol oder 100 %iges Propanol, wobei in den Proteinkristallen nur das intrakristalline Wasser (Kristallwasser) verbleibt, die Erzeugung eines Wirbelbettes möglich ist. Während der anschließenden Trocknungsphase konnte jedoch, auch nach längerer Trocknungszeit, das Trocknungsmedium nicht ausreichend entfernt werden. Die getrockneten Produkte zeigten einen Restgehalt an dem Trocknungsmedium von mehr als 5 %. Andererseits wurde während der Trocknungszeit bereits teilweise das Kristallwasser entfernt.

Es wurde nun überraschenderweise gefunden, daß nach einem Austausch des interkristallinen Wassers gegen ein Trocknungsmedium, das aus einer Mischung von Wasser und einer nicht-wäßrigen Substanz bestand, die erwähnten Nachteile vermieden werden konnten. Nach dem Austausch des interkristallinen Wassers gegen eines der unten beschriebenen Trocknungsmedien konnte aus dem abgesprengten Filterkuchen ein Wirbelbett erzeugt und das Kristallisat befriedigend getrocknet werden. Unter Verwendung von angefeuchtetem Trocknungsgas wurden nach einer Trocknungszeit von ca. 1 bis 4 Stunden Produkte erhalten, die frei von Kristallaggregaten waren und einen Restgehalt an den nicht-wäßrigen Substanzen von weniger als 0,1 % aufwiesen.

Demgemäß betrifft die vorliegende Erfindung ein Verfahren zur Trocknung von Proteinkristallen ausgehend von einer wäßrigen Proteinkristallsuspension, dadurch gekennzeichnet, daß die Proteinkristallsuspension in einem Zentrifugentrockner mit einem Trocknungsgas getrocknet wird, wobei das Trocknungsgas vor dem Trocknungsprozess mit Wasser angefeuchtet wird, und wobei die Proteinkristalle nach dem Abfiltrieren derselben aus der Proteinkristallsuspension in ein Trocknungsmedium verbracht werden, das aus einer Mischung von Wasser und einem nicht-wässrigen, mit Wasser in jedem Verhältnis mischbaren Lösungsmittel besteht, welches einen höheren Dampfdruck als Wasser besitzt.

Die nicht-wäßrigen Substanzen als Bestandteil der geeigneten Trocknungsmedien sind dadurch charakterisiert, daß sie bei einer Temperatur von ca. 40 °C einen niedrigen Dampfdruck besitzen (d.h. einen niedrigeren Dampfdruck als Wasser aufweisen), mit Wasser in jedem Verhältnis mischbar sind und unter den angegebenen Bedingungen gegen Proteine chemisch inaktiv sind. Alkohole wie z.B. Methanol, Ethanol, n-Propanol und Isopropanol erfüllen diese Bedingungen besonders gut. Auch Mischungen von Alkoholen sind geeignet.

Der Anteil der nicht-wäßrigen Substanzen in den geeigneten Mischungen mit Wasser in dem Trocknungsmedium beträgt von einschließlich etwa 10 % bis einschließlich etwa 80 %, vorzugsweise von einschließlich 15 % bis einschließlich etwa 60 %, weiterhin bevorzugt von einschließlich etwa 20 % bis einschließlich etwa 80%.

Bei dem beschriebenen Trocknungsverfahren unter Verwendung von trockenem Stickstoff als Trocknungsgas ist der Trocknungsprozeß bis zum Schluß kinetisch kontrolliert, so daß die oben beschriebenen Nachteile auftreten können.

Eigene Versuche unter Verwendung von feuchtem Stickstoff als Trocknungsgas haben gezeigt, daß die Entfernung des Wassers aus den Kristallen während der Trocknungsphase zum Stillstand kommt und der erreichte Rest-Wassergehalt in den getrockneten Kristallen von dem Wassergehalt im Trocknungsgas bestimmt wird. D.h. am Ende der Trocknungsphase stellt sich ein thermodynamisches Gleichgewicht zwischen dem Wassergehalt im Trocknungsgas und dem Wassergehalt in den Kristallen ein. Damit ist der Rest-Wassergehalt in den getrockneten Kristallen unabhängig von deren Größe (bzw. Oberflächengröße).

Außerdem haben die Versuche unter Verwendung von trockenem Stickstoff als Trocknungsgas gezeigt, daß der nicht-wäßrige Bestandteil des Trocknungsmediums, auch nach längerer Trocknungszeit, nicht ausreichend entfernt werden konnte. Die getrockneten Produkte zeigten einen Restgehalt an dem nicht-wäßrigen Bestandteil von mehr als 1 %.

Es wurde überraschenderweise gefunden, daß bei einem Verfahren zur Trocknung von Proteinkristallen unter Verwendung eines Trocknungsgases der Restgehalt an dem nicht-wäßrigen Bestandteil des Trocknungsmediums bis auf weniger als 0,1 % entfernt werden konnte, wenn ein wasserhaltiges Trocknungsgas eingesetzt wird. Das Trocknungsverfahren wird vorzugsweise in einem Zentrifungentrockner durchgeführt. Besonders vorteilhaft ist es bei dem Verfahren zur Trocknung von Proteinkristallen, wenn die Proteinkristalle nach dem Abfiltrieren derselben aus der Proteinkristallsuspension in ein Trocknungsmedium verbracht werden, das aus einer Mischung von Wasser und einem nicht-wäßrigen, mit Wasser in jedem Verhältnis mischbaren Lösungsmittel besteht, welches einen niedrigeren Dampfdruck als Wasser besitzt. Besonders vorteilhaft ist es dabei, wenn die Proteinkristalle nach dem Abfiltrieren derselben aus der Proteinkristallsuspension zum Zweck der Trocknung in ein Wirbelbett überführt werden.

Eigene Versuche mit dem beschriebenen Trocknungsverfahren haben gezeigt, daß bei dem Einsatz einer sterilen und von Fremdpartikeln freien Kristallsuspension und der Sterilfiltration von Waschwasser, Trocknungsmedium und Trocknungsgas sowie nach einer geeigneten Reinigung und Sterilisation des Zentrifugentrockners ein getrocknetes Produkt erhalten wurde, das sowohl keimfrei als auch frei von Fremdpartikeln war.

Das Verfahren gemäß der vorliegenden Erfindung ist besonders zur Trocknung von Kristallen von tierischem Insulin, Humaninsulin oder einem Analogon derselben geeignet.

Als Insulinanaloga werden hier Derivate von natürlich vorkommenden Insulinen, nämlich Humaninsulin oder tierischen Insulinen, bezeichnet, welche sich durch Substitution wenigstens eines natürlich auftretenden Aminosäurerestes und/oder Addition wenigstens eines Aminosäurerestes und/oder organischen Restes von dem entsprechenden, ansonst gleichen natürlich vorkommenden Insulin unterscheiden.

Im folgenden soll das erfindungsgemäße Verfahren, insbesondere anhand von Beispielen näher erläutert werden.

### Aufbau und Wirkungsweise des Zentrifugentrockners:

Der Zentrifugentrockner besteht aus einer waagerecht angeordneten Zentrifugentrommel mit einem kompakten Mantel. In der Trommel befindet sich ein zylindrischer Siebkorb, der mit der Trommel fest verbunden ist. Der Raum zwischen der porösen Mantelfläche des Siebkorbes und der kompakten Mantelfläche der Trommel ist, radial in mehrere Kammem unterteilt. In der hinteren Stimfläche der Trommel befinden sich Öffnungen, so daß während der Rotation der Trommel entweder von außen ein Gas (z.B. das Trocknungsgas) in die Kammem geleitet werden kann oder eine Flüssigkeit (z.B. die Mutterlauge der Kristallsuspension) aus den Kammem nach außen abgeleitet werden kann. Die Trommel ist vom mit einem Stempel verschlossen, der mit der Trommel rotiert. Bei ruhender Trommel kann der Stempel in axialer Richtung bewegt werden. Wenn der Stempel zurückgefahren wird, wird eine feststehende kreisförmige Auslaßbahn freigegeben, die dann vom Innenraum der Zentrifugentrommel zugänglich ist. Nachdem die Trommel geöffnet worden ist, kann die Trommel wieder langsam rotieren (um das trockene Produkt auszutragen).

Bei dem Zentrifugentrockner, der in den Versuchen verwendet wurde, hatte die Zentrifugentrommel einen Durchmesser von 400 mm und eine Mantellänge von 200 mm, der Siebkorb hatte eine Filterfläche von 0,25 m² und eine Porengröße von 10 µm.

Die Zentrifuge wird bei drehender Trommel (475 UpM) axial durch die hohle Antriebswelle mit Produkt (Kristallsuspension) beschickt, wobei sich der Filterkuchen (Kristallisat) als Schicht auf dem Siebmantel ausbildet und das Filtrat durch den Siebmantel in die Kammem und von dort durch den Trommelboden in den feststehenden Außenraum gedrückt wird. Das Filtrat (Mutterlauge der Kristallisation) verläßt die Zentrifuge durch den Auslaßstutzen und wird verworfen. Anschließend wird der Filterkuchen auf dem gleichen Wege mit Wasser gewaschen. Danach wird das Waschwasser auf dem gleichen Wege gegen eines der in den Beispielen genannten Trocknungsmedien ausgetauscht. Nachdem man den Filterkuchen bei höherer Drehzahl (1.200 UpM) trockengeschleudert hat, wird bei niederer Drehzahl (6 UpM) durch Einblasen von Stickstoff der Filterkuchen vom Siebmantel abgesprengt und das abgesprengte Produkt in ein Wirbelbett überführt und getrocknet. Dazu wird ein pulsierender Stickstoffstrom von außen (durch die Öffnungen im Trommelboden) durch die unteren Trommeikammern und durch den unteren Teil des Siebmantels in den Innenraum der Trommel geleitet. Das Trocknungsgas durchströmt das rotierende Wirbelbett des Produktes und verläßt die Trommel über den oberen Teil des Siebmantels und die obere Trommelkammer. Nach Beendigung der Trocknungsphase wird der Stempel zurückgefahren, so daß die kreisförmige Auslaufbahn freigegeben wird. Bei niedriger Drehzahl und pulsierenden Druckstößen schleudert der Stickstoff das getrocknete Produkt in die Auslaßbahn. Am Ende der Auslaßbahn wird der Stickstoff in einem Zyklon abgeschieden und das trockene Produkt in einem Vorratsgefäß aufgefangen.

Das Trocknungsgas (Stickstoff) wird vor dem Eintritt in den Zentrifugentrockner in einer separaten Apparatur mit sterilem Wasserdampf befeuchtet und auf die Trocknungstemperatur erwärmt. Die Temperatur und der Wassergehalt im Stickstoffstrom werden geregelt.

### 1. Beispiel:

In einem Kristallisationskessel mit Rührer wurden 1000 g Insulin vom Schwein aus wäßriger Lösung bei pH 5,5 unter Zusatz von Zinkionen kristallisiert. Nach Beendigung der Kristallisation enthielt die Suspension (ca. 100 L) rhomboedrische Kristalle mit einer mittleren Teilchengröße von 25 µm. Wie im allgemeinen Teil beschrieben, wurde die gerührte Kristallsuspension innerhalb von ca. 30 Min. in den Zentrifugentrockner gegeben. Der Filterkuchen wurde mit ca. 50 L Wasser gewaschen und danach das Waschwasser gegen 70 %iges wäßriges Ethanol (Trocknungsmedium) ausgetauscht. Nachdem der Filterkuchen vom Siebmantel abgesprengt war, wurde das Kristallisat im Wirbelbett getrocknet. Während der Trocknungszeit war das Trocknungsgas auf eine Temperatur von 40 °C vorgewärmt und auf einen Wassergehalt von 4 g Wasser pro 1 Kg Stickstoff eingestellt worden. Nach 120 Min. war die Trocknung beendet und das Produkt wurde in ein Vorratsgefäß entleert. Die Analyse des trocknen Kristallisats zeigte eine Restfeuchte an Wasser von 5 % und eine Restgehalt an Ethanol von weniger als 0,1 %.

### 2. Beispiel:

In einem Kristallisationskessel mit Rührer wurden unter sterilen Bedingungen 1500 g Humaninsulin aus wäßriger Lösung bei pH 5,5 unter Zusatz von Zinkionen kristallisiert. Nach Beendigung der Kristallisation enthielt die Suspension (ca. 150 L) rhomboedrische Kristalle mit einer mittleren Teilchengrüße von 20 µm. Der Zentrifugentrockner wurde vor dem Einsatz nacheinander mit 10 %iger Natronlauge, partikelfreiem Wasser, 10 %iger Essigsäure und erneut mit partikelfreiem Wasser gewaschen. Anschließend wurde der Zentrifugentrockner mit partikelfreiem Dampf sterilisiert. Wie im allgemeinen Teil beschrieben, wurde die gerührte Kristallsuspension innerhalb von ca. 45 Min. in den Zentrifugentrockner gegeben. Der Filterkuchen wurde mit ca. 50 L Wasser gewaschen und danach das Waschwasser gegen 50 %iges wäßriges Ethanol (Trocknungsmedium) ausgetauscht. Beide Flüssigkeiten waren zuvor sterilfiltriert worden. Nachdem der Filterkuchen vom Siebmantel abgesprengt war, wurde das Kristallisat im Wirbelbett getrocknet. Während der Trocknungszeit war das sterilfiltrierte Trocknungsgas auf eine Temperatur von 40 °C vorgewärmt und auf einen Wassergehalt von 3 g Wasser pro 1 Kg Stickstoff eingestellt worden. Nach 150 Min. war die Trocknung beendet und das Produkt wurde, wie oben beschrieben, in ein Vorratsgefäß entleert. Die Analyse des trocknen Kristallisats zeigte eine Restfeuchte an Wasser von 4 % und eine Restgehalt an Ethanol von weniger als 0,1 %. Das Produkt war frei von Keimen und Fremdpartikeln.

### 3. Beispiel:

In einem Kristallisationskessel mit Rührer wurden 1000 g Humaninsulin aus wäßriger Lösung bei pH 5,5 unter Zusatz von Zinkionen kristallisiert. Nach Beendigung der Kristallisation enthielt die Suspension (ca. 100 L) rhomboedrische Kristalle mit einer mittleren Teilchengröße von 25 µm. Wie im allgemeinen Teil beschrieben, wurde die gerührte Kristallsuspension innerhalb von ca. 30 Min. in den Zentrifugentrockners gegeben. Der Filterkuchen wurde mit ca. 50 L Wasser gewaschen und das Waschwasser gegen 30 %iges wäßriges Ethanol (Trocknungsmedium) ausgetauscht. Nachdem der Filterkuchen vom Siebmantel abgesprengt war, wurde das Produkt im Wirbelbett getrocknet. Während der Trocknungszeit war das Trocknungsgas auf eine Temperatur von 40 °C vorgewärmt und auf einen Wassergehalt von 5 g Wasser pro 1 Kg Stickstoff eingestellt worden. Nach 120 Min. war die Trocknung beendet und das Produkt wurde in ein Vorratsgefäß entleert. Die Analyse des trocknen Kristallisats zeigte eine Restgehalt an Wasser von 6 % und eine Restgehalt an Ethanol von weniger als 0,1 %.

### 4. Beispiel:

In einem Kristallisationskessel mit Rührer wurden 1000 g Insulin vom Schwein aus wäßriger Lösung bei pH 5,5 unter Zusatz von Zinkionen kristallisiert. Nach Beendigung der Kristallisation enthielt die Suspension (ca. 100 L) rhomboedrische Kristalle mit einer mittleren Teilchengröße von 30 µm. Wie im allgemeinen Teil beschrieben, wurde die gerührte Kristallsuspension innerhalb von ca. 30 Min. in den Zentrifugentrockners gegeben. Der Filterkuchen wurde mit ca. 50 L Wasser gewaschen und das Waschwasser gegen 72 %iges wäßriges Propanol (Trocknungsmedium) ausgetauscht. Nachdem der Filterkuchen vom Siebmantel abgesprengt war, wurde das Produkt im Wirbelbett getrocknet. Während der Trocknungszeit war das Trocknungsgas auf eine Temperatur von 40 °C vorgewärmt und auf einen Wassergehalt von 4 g Wasser pro 1 Kg Stickstoff eingestellt worden. Nach 140 Min. war die Trocknung beendet und das Produkt wurde in ein Vorratsgefäß entleert. Die Analyse des trocknen Kristallisats zeigte eine Restfeuchte an Wasser von 5 % und eine Restgehalt an Propanol von weniger als 0,1 %.

### 5. Beispiel:

In einem Kristallisationskessel mit Rührer wurden 1500 g Di-Arg-Insulin aus wäßriger Lösung bei pH 6,3 unter Zusatz von Zinkionen kristallisiert. Nach Beendigung der Kristallisation enthielt die Suspension (ca. 100 L) Kristalle mit einer mittleren Teilchengröße von 20 µm. Wie im allgemeinen Teil beschrieben, wurde die gerührte Kristallsuspension innerhalb von ca. 30 Min. in den Zentrifugentrockners gegeben. Der Filterkuchen wurde mit ca. 50 L Wasser gewaschen und das Waschwasser gegen 25 %iges wäßriges Propanol (Trocknungsmedium) ausgetauscht. Nachdem der Filterkuchen vom Siebmantel abgesprengt war, wurde das Produkt im Wirbelbett getrocknet. Während der Trocknungszeit war das Trocknungsgas auf eine Temperatur von 40 °C vorgewärmt und auf einen Wassergehalt von 3 g Wasser pro 1 Kg Stickstoff eingestellt worden. Nach 160 Min. war die Trocknung beendet und das Produkt wurde in ein Vorratsgefäß entleert. Die Analyse des trocknen Kristallisats zeigte eine Restfeuchte an Wasser von 6 % und eine Restgehalt an Propanol von weniger als 0,1 %.

### 6. Beispiel:

In einem Kristallisationskessel mit Rührer wurden 1000 g Humaninsulin aus wäßriger Lösung bei pH 5,5 unter Zusatz von Zinkionen kristallisiert. Nach Beendigung der Kristallisation enthielt die Suspension (ca. 100 L) rhomboedrische Kristalle mit einer mittleren Teilchengröße von 25 µm. Wie im allgemeinen Teil beschrieben, wurde die gerührte Kristallsuspension innerhalb von ca. 30 Min. in den Zentrifugentrockners gegeben. Der Filterkuchen wurde mit ca. 50 L Wasser gewaschen und das Waschwasser gegen 15 %iges wäßriges n-Propanol (Trocknungsmedium) ausgetauscht. Nachdem der Filterkuchen vom Siebmantel abgesprengt war, wurde das Produkt im Wirbelbett getrocknet. Während der Trocknungszeit war das Trocknungsgas auf eine Temperatur von 40 °C vorgewärmt. Der Wassergehalt im Trocknungsgas wurde zu Beginn der Trocknungszeit auf 15 % eingestellt und während der Trocknungszeit auf 8 % abgesenkt. Nach 120 Min. war die Trocknung beendet und das Produkt wurde in ein Vorratsgefäß entleert. Die Analyse des trocknen Kristallisats zeigte eine Restfeuchte an Wasser von 6 % und eine Restgehalt an Propanol von weniger als 0,1 %.

## Patentansprüche

1. Verfahren zur Trocknung von Proteinkristallen ausgehend von einer wässrigen Proteinkristallsuspension, **dadurch gekennzeichnet, dass** die Proteinkristallsuspension in einem Zentrifugentrockner mit einem Trocknungsgas getrocknet wird, wobei das Trocknungsgas vor dem Trocknungsprozess mit Wasser angefeuchtet wird, und wobei die Proteinkristalle nach dem Abfiltrieren derselben aus der Proteinkristallsuspension in ein Trocknungsmedium verbracht werden, das aus einer Mischung von Wasser und einem nicht-wässrigen, mit Wasser in jedem Verhältnis mischbaren Lösungsmittel besteht, welches einen höheren Dampfdruck als Wasser besitzt.

2. Verfahren nach Anspruch 1, **dadurch gekennzeichnet, dass** der Anteil des Lösungsmittels im Trocknungsmedium von einschließlich 10 bis einschließlich 80% beträgt.

3. Verfahren nach Anspruch 2, **dadurch gekennzeichnet, dass** der Anteil des Lösungsmittels im Trocknungsmedium von einschließlich 15 bis einschließlich 60% beträgt.

4. Verfahren nach einem oder mehreren der Ansprüche 1 bis 3, **dadurch gekennzeichnet, dass** das Lösungsmittel ein Alkohol oder ein Gemisch von Alkoholen ist.

5. Verfahren nach Anspruch 4, **dadurch gekennzeichnet, dass** das Lösungsmittel Methanol ist.

6. Verfahren nach Anspruch 4 oder 5, **dadurch gekennzeichnet, dass** das Lösungsmittel Ethanol ist.

7. Verfahren nach einem oder mehreren der Ansprüche 4 bis 6, **dadurch gekennzeichnet, dass** das Lösungsmittel n-Propanol ist.

8. Verfahren nach einem oder mehreren der Ansprüche 4 bis 7, **dadurch gekennzeichnet, dass** das Lösungsmittel iso-Propanol ist.

9. Verfahren nach einem der mehreren derAnsprüche 1 bis 8, **dadurch gekennzeichnet, daß** das Trocknungsgas Stickstoff ist.

10. Verfahren zur Trocknung von Proteinkristallen ausgehend von einer wässrigen Proteinkristallsuspension nach einem oder mehreren der Ansprüche 1 bis 9, **dadurch gekennzeichnet, dass** die Proteinkristalle nach dem Abfiltrieren derselben aus der Proteinkristallsuspension zum Zweck der Trocknung in ein Wirbelbett überführt werden.

11. Verfahren nach einem oder mehreren der Ansprüche 1 bis 10, **dadurch gekennzeichnet, dass** die Proteinkristalle Kristalle von tierischem Insulin, Humaninsulin oder einem Analogon derselben sind.

## Claims

1. Process for drying protein crystals starting from an aqueous protein crystal suspension, **characterized in that** the protein crystal suspension is dried using a drying gas in a centrifugal dryer, where the drying gas is moistened with water before the drying process and where the protein crystals, after they have been filtered off from the protein crystal suspension, are brought into a drying medium which consists of a mixture of water and a nonaqueous solvent which is miscible with water in any ratio and which has a higher vapour pressure than water.

2. Process according to Claim 1, **characterized in that** the proportion of the solvent in the drying medium is from 10 to 80% inclusive.

3. Process according to Claim 2, **characterized in that** the proportion of the solvent in the drying medium is from 15 to 60% inclusive.

4. Process according to one or more of Claims 1 to 3, **characterized in that** the solvent is an alcohol or a mixture of alcohols.

5. Process according to Claim 4, **characterized in that** the solvent is methanol.

6. Process according to Claim 4 or 5, **characterized in that** the solvent is ethanol.

7. Process according to one or more of Claims 4 to 6, **characterized in that** the solvent is n-propanol.

8. Process according to one or more of Claims 4 to 7, **characterized in that** the solvent is isopropanol.

9. Process according to one or more of Claims 1 to 8, **characterized in that** the drying gas is nitrogen.

10. Process for drying protein crystals starting from an aqueous protein crystal suspension according to one or more of Claims 1 to 9, **characterized in that** the protein crystals, after they have been filtered off from the protein crystal suspension, are converted into a fluidized bed for the purpose of drying.

11. Process according to one or more of Claims 1 to 10, **characterized in that** the protein crystals are crystals of animal insulin, human insulin or an analogue thereof.

## Revendications

1. Procédé pour le séchage de cristaux protéiniques, partant d'une suspension aqueuse de cristaux protéiniques, **caractérisé en ce que** la suspension de cristaux protéiniques est séchée dans un sécheur centrifuge avec un gaz de séchage, le gaz de séchage étant humidifié avant le processus de séchage avec de l'eau et les cristaux protéiniques étant introduits, après leur séparation par filtration de la suspension de cristaux protéiniques, dans un milieu de séchage qui est constitué par un mélange d'eau et d'un solvant non aqueux, miscible à l'eau dans tous les rapports, qui présente une tension de vapeur supérieure à celle de l'eau.

2. Procédé selon la revendication 1, **caractérisé en ce que** la proportion de solvant dans le milieu de séchage est de 10 compris, jusqu'à et y compris 80%.

3. Procédé selon la revendication 2, **caractérisé en ce que** la proportion de solvant dans le milieu de séchage est de 15 compris, jusqu'à et y compris 60%.

4. Procédé selon l'une ou plusieurs des revendications 1 à 3, **caractérisé en ce que** le solvant est un alcool ou un mélange d'alcools.

5. Procédé selon la revendication 4, **caractérisé en ce que** le solvant est le méthanol.

6. Procédé selon la revendication 4 ou 5, **caractérisé en ce que** le solvant est l'éthanol.

7. Procédé selon l'une ou plusieurs des revendications 4 à 6, **caractérisé en ce que** le solvant est le n-propanol.

8. Procédé selon l'une ou plusieurs des revendications 4 à 7, **caractérisé en ce que** le solvant est l'iso-propanol.

9. Procédé selon l'une ou plusieurs des revendications 1 à 8, **caractérisé en ce que** le gaz de séchage est l'azote.

10. Procédé pour le séchage de cristaux protéiniques partant d'une suspension aqueuse de cristaux protéiniques selon l'une ou plusieurs des revendications 1 à 9, **caractérisé en ce que** les cristaux protéiniques sont transférés après leur séparation par filtration de la suspension de cristaux protéiniques dans un lit tourbillonnant en vue du séchage.

11. Procédé selon l'une ou plusieurs des revendications 1 à 10, **caractérisé en ce que** les cristaux protéiniques sont des cristaux d'insuline animale, d'insuline humaine ou d'un analogue de celles-ci.
